# EUROPEAN PATENT APPLICATION

(11) **EP 0 759 476 A1**
(43) Date of publication of application: **26.02.1997**
(21) Application number: 95917499.6
(22) Date of filing: 02.05.1995
(51) Int. Cl.: C12Q 1/68

(54) **METHOD OF DETECTING HUMAN CYTOCHROME P4502D6 GENE POLYMORPHISM**

(30) Priority: 06.05.1994 JP 94398/94
(71) Applicant: OTSUKA PHARMACEUTICAL CO., LTD., Chiyoda-ku Tokyo 101 (JP)
(72) Inventor: KAMATAKI, Tetsuya Minamihachijo-jutaku 401-14, Sapporo-shi Hokkaido 064 (JP)
(74) Representative: Kolb, Helga, Dr. Dipl.-Chem.
(86) International application number: JP9500861
(87) International publication number: WO9530772

(57) **Abstract**

The present invention provides a method of detecting a polymorph of the human cytochrome P450 2D6 gene which comprises detecting the occurrence, in exon 9 of the human cytochrome P450 2D6 (CYP2D6) gene, of an insertion of a repetition of the 9 bases TCACCCGTG.

According to the method of the present invention, it is possible to detect a novel CYP2D6 polymorph that has not yet been reported.

## Description

### TECHNICAL FIELD

The present invention relates to a method of diagnosing genes for drug metabolism-associated enzymes and, more particularly, to a novel method of detecting a polymorph of the cytochrome P450 2D6 (hereinafter abbreviated as CYP2D6) gene.

### BACKGROUND ART

Human cytochrome P450 (P450) is an enzyme performing such important functions as detoxication and metabolic activation of drugs or exogenous materials and biosynthesis of steroid hormones and bile acid. CYP2D6 is one of the molecular species of said P450 and is involved in particular in the metabolism of a number of drugs of clinical therapeutic importance. So far, as many as 26 kinds of drugs such as the antihypertensives debrisoquin and propafenone, the β-blocker propranolol, the tricyclic antidepressant imipramine, etc. have been reported to serve as specific substrates for said CYP2D6 [Eichelbaum, M, and Gross, A. S., Clin. Pharmac. Ther., 46, 377-394 (1990)].

On the other hand, it has been revealed that polymorphism exists of said CYP2D6. It is reported that in poor metabolizers (hereinafter abbreviated as PMs), a drug administered at a usual dose may produce an excessive effect (unwanted effect) because of a high blood level of the drug being maintained in them. The polymorphism of CYP2D6 differs among races and the percentage of PMs among Europeans and Americans is 5 to 10% [Kimura, S., Umeno, M., Skoda, R. C., Meyer, U. A. and Gonzalez, F. J., Am. J. Hum. Genet., 45, 889-904 (1989)] while, among Japanese, the percentage is as low as 0 to 2% [Yokota, T., Tamura, S., Furuya, H., Kimura, S., Watanabe, M. Kanazawa, I., Kondo, I. and Gonzalez, F. J., Pharmacogenetics 3, 256-263 (1993)]. Therefore, if a new drug prior to approval is a specific substrate for CYP2D6, it it quite possible that some serious adverse effect be overlooked in the stage of clinical investigation because of its low frequency among PMs but, after marketing, manifest itself. As far as Japanese are concerned, particular care should be taken in this respect [Kamataki, Tetsuya, TDM Kenkyu, 10, 2-7 (1993)].

Gene analyses recently made concerning CYP2D6 in Europeans and Americans revealed that mutations in the CYP2D6 gene are the causes of the above-mentioned polymorphism of CYP2D6 [Gonzalez, F. J. and Meyer, U. A., Clin. Pharmac. Ther., 50, 233-238 (1991); Heim, M. and Meyer, U. A., Lancet, 336, 529-532 (1990); Smith, C. A. D., Gough, A. C., Leigh, P. N., Summers, B. A., Harding, A. E., Maranganore, D. M., Sturman, S. G., Schapira, A. H. V., Williams, A. C., Spurr, N. K. and Wolf, C. R., Lancet, 339, 1373-1377 (1992)]. Thus, while the CYP2D6 gene consists of 9 exons, there are reportedly three mutations, namely type A mutation consisting in one base substitution in exon 5 at 2637th position of the genomic DNA, type B mutation consisting in one base substitution in exon 4 at the exon-intron junction of 1934th position and type D mutation consisting in complete deletion. It has been revealed that these mutations and deletion result in a marked impairment of the metabolic ability.

Among the CYP2D6 mutations mentioned above, the type A and type B mutations estimably account for a very high percentage. According to a report, 95% of PMs among Europeans and Americans can be explained in terms of both the mutations mentioned above [Daly, A. K., Armstrong, M., Monkman, S. C., Idle, M. E. and Idle, J. K., Pharmacogenetics, 1, 33-41 (1991)]. Another report says that 75% of PMs among Europeans and Americans fall under the type B mutation and 5 to 11% under the type A mutation [Gonzalez, F. J., et al., op. cit.].

As regards Japanese, however, the frequencies of these mutations are so low that the relevant studies have little advances.

### DISCLOSURE OF THE INVENTION

The present invention reveals a novel mutation of the CYP2D6 gene and it is an object of the present invention to provide a novel method of detecting such a polymorph of the CYP2D6 gene. In particular, it is an object of the present invention to develop a detection method of the above kind which is simple and expedient and can detect a novel form involved in the polymorphism of the CYP2D6 gene with high sensitivity and accuracy, using small amounts of DNA samples.

To attain the above objects, the present inventors made an analysis of the CYP2D6 gene in Japanese PMs as well as a genealogical analysis. As a result, they discovered a novel gene polymorph useful for the above objects and succeeded in developing a detection technology therefor, and have now completed the present invention.

Thus, the present invention is concerned with a method of detecting one of the polymorphic forms of the CYP2D6 gene which comprises detecting the occurrence, in exon 9 of the CYP2D6 gene, of an inserted sequence composed of a repetition of the nine base subsequence TCACCCGTG.

In the present specification, the amino acid and base sequences are represented in terms of those abbreviations that are recommended by the IUPAC-IUB or are in common or idiomatic use in the relevant field of art and the bases are numbered in accordance with the CYP2D6 genomic DNA [the notation by Yokota, T., et al., op. cit.].

The novel type of mutation as revealed by the present invention is characterized by the occurrence of an inserted sequence composed of a repetition of the nine base subsequence TCACCCGTG in the 4213th position, namely in exon 9, of the CYP2D6 gene.

The method of the present invention is not limited to any particular technique or means provided that such particular mutation can be detected thereby. Thus, any of various methods and techniques which are conventional can widely be employed. Since the gene mutation to be detected has thus been identified and specified by the present invention, it will be easy to the one skilled in the art to select an adequate technique for the detection thereof in accordance with the disclosure of the present application. For instance, the method of the present invention may consist in, and of course includes, the base sequence analysis at the site specified above. The Southern hybridization technique or dot hybridization technique [for each technique: Southern, E. M., J. Mol. Biol., 98, 503-517 (1975)] may also possibly be a proper choice. For example, the PCR (polymerase chain reaction)-RFLP (restriction fragment length polymorphism) method, the PCR-single strand conformation polymorphism method [Orita, M., Iwahana, H., Kanazawa, H., Hayashi, K. and Sekiya, T., Proc. Natl. Acad. Sci., U. S. A., 86, 2766-2770 (1989)], the PCR-SSO (specific sequence oligonucleotide) method, and the ASO (allele specific oligomer) method using the PCR-SSO and dot hybridization techniques [Saiki, R. K., Bugawan, T. L., Horn, G. T., Mullis, K. B. and Erlich, H. A., Nature, 324, 163-166 (1986)], among others, can be followed.

In particular, the above-mentioned RFLP technique may be mentioned as a preferred one to be employed in the practice of the present invention and, particularly when said technique is combined with the DNA amplification technique utilizing the PCR, it is possible to detecting said polymorph in a simple and easy manner with high sensitivity and accuracy using only small amounts of DNA samples. Hereinafter, taking this detection method (PCR-RFLP method) as an example, the present invention is described in further detail.

Various procedures employable in the detection method according to the present invention, for example chemical synthesis of certain DNAs, enzymatic treatment for DNA cleavage, deletion, addition or ligation, DNA isolation, purification, replication, selection and so forth, can each be carried out in the conventional manner [e.g. Bunshi Idengaku Jikkenho (Experiments in Molecular Genetics), published 1983 by Kyoritsu Shuppan; PCR Technology, published 1990 by Takara Shuzo]. Thus, for instance, DNA can be isolated and purified by agarose gel electrophoresis or the like, and DNA can be sequenced by the dideoxy method [Sanger, F., Nicklen, S. and Coulson, A. R., Proc. Natl. Acad. Sci., U.S.A., 74, 5463-5467 (1977)], the Maxam-Gilbert method [Maxam, A. M. and Gilbert, W., Methods in Enzymology, 65, 499-560 (1980)], etc. Said DNA base sequence determination may also be readily performed by using commercially available sequencing kits or the like. The PCR for amplifying a certain specific region of DNA, too, can be performed in the conventional manner [e.g. Saiki, R. K., Scharf, S., Faloona, F. A., Mullis, K. B., Horn, G. T., Erlich, H. A. and Arnheim, N., Science, 230, 1350-1354 (1985)]. These various basic procedures are also employed in various references cited herein and reference is hereby made thereto together with the examples mentioned later herein.

As for the genomic DNA to be subjected to analysis by the detection method of the present invention, any human-derived sample containing said DNA can be employed without any particular limitation. As examples, there may be mentioned body fluids such as blood, bone marrow fluid, semen and peritoneal fluid, liver and other tissue cells, and body hair such as hair on the head, among others. Genomic DNA can be prepared by extracting and purifying from these samples by the conventional method. Concentrated test samples can be obtained in large quantities by amplifying, based on said genomic DNA, a certain DNA region containing the site of mutation with which the present invention is concerned. This is realized, for example by the PCR technique, employing primers adequately designed so that the CYP2D6 gene alone, inclusive of the above-mentioned inserted portion in exon 9, can be specifically amplified. Such primers can be designed by the conventional method. The base length of the region to be amplified, for instance, is not critical but, generally, it may have a length of about 100 bp to about 500 bp. The DNA region to be amplified is selected so that it contains an arbitrary restriction enzyme site which can suitably be used in the subsequent RFLP step. Said region can be arbitrarily selected provided that the cleavage fragment obtained upon treatment of the DNA region with said enzyme can provide a difference in length which enables the detection by comparison between the mutant gene and the normal (wild) gene. A preferred example of such primer designing is shown later herein in an example. The primers are a sense primer (9-S) and an antisense primer (9-AS) and such designing makes it possible to specifically amplify the above-mentioned desired region of CYP2D6 alone in distinction from the two pseudogenes CYP2D7 and CYP2D8 highly homologous in base sequence to CYP2D6. By designing such primers, desired DNA regions having a length of 334 bp (wild) or 343 bp (mutant) can be amplified, which contain a desired unique restriction enzyme site (StyI site) and, upon treatment with the enzyme, give a cleavage fragment of 77 bp (wild) or 86 bp (mutant) and a common fragment of 257 bp.

The desired DNA region thus amplified by PCR can readily be tested for detection and identification of the mutation contained therein by employing the Southern blot or gel electrophoresis technique, for instance, for RFLP detection. Said desired DNA region is subjected to treatment (digestion) with the above-mentioned adequately selected restriction enzyme and the cleavage fragments formed are identified as specific bands by the conventional method.

Based on the pattern of the bands obtained in the above manner, the CYP2D6 gene polymorph (occurrence of the mutation revealed by the present inventors) can be detected.

### BRIEF DESCRIPTION OF THE DRAWINGS

Fig. 1 shows the base sequences and occurring sites of the primers designed and constructed in Reference Example 2 (1).

Fig. 2 shows the base sequences and occurring sites of the primers designed and constructed in Example 1 (1).

Fig. 3 shows an electrophoretogram obtained by the method shown in Example 1 for a PM and an EM as well as the bases of the 9-base insertion observed as a result.

Fig. 4 shows the base sequences and the sites of occurrence of the primers designed and constructed in Example 2 (1).

Fig. 5 illustrates the judgment made in Example 2 (3) based on the band length of the insertion in exon 9 of the CYP2D6 gene.

Fig. 6 is a schematic representation of the distinction among the types judged according to the principle shown in Fig. 5.

Fig. 7 shows the results obtained for a PM and the parents thereof according to Example 2.

### BEST MODES FOR CARRYING OUT THE INVENTION

The following examples illustrate the present invention in further detail but are by no means limitative of the scope thereof.

The abbreviations listed below are used for the reagents and other materials used in the respective examples. The compositions are given in terms of concentrations or amounts per liter. When necessary, some materials were sterilized by autoclaving (121°C, 20 minutes) etc.
- TE solution ··· 10 mM Tris-HCl (pH 7.5), 1 mM ethylene-diaminetetraacetic acid disodium salt (EDTA) (pH 8.0);
- 20 x SSC ··· 3 M sodium chloride, 0.3 M sodium citrate;
- 5 x Denhardt's solution ··· 10 g Ficoll, 10 g polyvinyl-pyrrolidone, 10 g bovine serum albumin (BSA);
- 2 x Hybridization buffer (pH 8.0) ··· 0.1 M Tris-HCl (pH 8.0), 2 M sodium chloride, 20 mM EDTA, 0.2% sodium dodecyl sulfate (SDS);
- SE solution ··· 10 mM Tris-HCl (pH 7.4), 50 mM EDTA, 0.5% N-lauroylsarcosine disodium salt;
- Fromamide dye ··· 100 ml formamide, 0.1 g xylene cyanol FF, 0.1 g bromophenol blue, 4 ml 0.5 M EDTA;
- STE ··· 100 mM sodium chloride, 10 mM Tris-HCl (pH 7.5), 1 mM EDTA (pH 8.0);
- LB medium ··· 1 g glucose, 5 g sodium chloride, 5 g yeast extract, 10 g Bacto-tryptone;
- LBAmp⁺ medium ··· 1 g glucose, 5 g sodium chloride, 5 g yeast extract, 10 g Bacto-tryptone, ampicillin (added, after autoclaving, to a final concentration of 25 µg/ml);
- LBAmp⁺ plate ··· the above LBAmp⁺ medium supplemented with 11 g of agar;
- 2 x TY medium ··· 16 g Bacto-tryptone, 10 g yeast extract, 5 g sodium chloride;
- 2 x TY plate ··· the above 2 x TY medium supplemented with 11 g of agar;
- 10 x PCR buffer (pH 8.3) ··· 100 mM Tris-HCl (pH 8.3), 500 mM potassium chloride, 0.01% (w/v) gelatin, magnesium chloride (concentration thereof specified in each occasion in mM);
- H agar medium ··· 20 g Bacto-tryptone, 8 g sodium chloride, 12 g agar powder;
- H top layer agar ··· 20 g Bacto-tryptone, 8 g sodium chloride, 8 g agar powder;
- 10 x T4 DNA polymerase buffer (pH 8.8) ··· 667 mM Tris-HCl (pH 8.8), 67 mM magnesium chloride, 116 mM ammonium sulfate, 100 mM 2-mercaptoethanol, 67 µM EDTA, 0.167% BSA;
- 10 x Denaturation buffer (pH 9.5) ··· 20 mM Tris-HCl (pH 9.5), 1 mM spermidine, 0.1 mM EDTA;
- 10 x Blunt end buffer ··· 0.5 M Tris-HCl (pH 9.5), 0.1 M magnesium chloride, 0.1 M 2-mercaptoethanol;
- Labeling mix ··· 7.5 µM deoxyguanosine 5'-triphosphate (dGTP), 7.5 µM deoxycyitidine 5'-triphosphate (dCTP), 7.5 µM deoxythymidine 5'-triphosphate (dTTP);
- 40% Acrylamide stock solution ··· 38 g acrylamide, 2 g bisacrylamide, purified water to make a total volume of 100 ml;
- 10 x TBE (pH 8.3) ··· 108 g Tris, 55 g boric acid, 9.3 g EDTA;
- Gel A ··· 76.8 g urea, 24 ml 40% acrylamide stock solution, 8 ml 10 x TBE, purified water to make a total volume of 160 ml;
- Gel B ··· 14.4 g urea, 4.5 ml 40% acrylamide stock solution, 7.5 ml 10 x TBE, 3.0 g sucrose, bromophenol blue dye solution (10 mg/ml), purified water to make a total volume of 30 ml;
- 10 x H solution ··· 500 mM Tris-HCl (pH 7.5), 100 mM magnesium chloride, 10 mM dithiothreitol (DTT), 100 mM sodium chloride;
- 10 x M solution ··· 100 mM Tris-HCl (pH 7.5), 100 mM magnesium chloride, 10 mM DTT, 500 mM sodium chloride.

### Reference Example 1

### (1) Preparation of human peripheral blood genomic DNA

Genomic DNA was prepared from human peripheral blood in the following manner. Thus, 10 ml of human peripheral blood collected using the anticoagulant heparin was centrifuged at 3,000 rpm for 10 minutes to give a buffy coat. The same volume of a cold PBS was added to the buffy coat obtained and the same centrifugation procedure was repeated 2 or 3 times. The buffer coat obtained was washed with 5 volumes of 0.2% sodium chloride-5 mM EDTA and centrifuged at 5,000 rpm for 10 minutes, and the sediment was recovered. The washing procedure mentioned above was repeated until the hemoglobin color of the sediment disappeared. TE solution (0.5 ml) was added to the sediment and, after attaining complete suspension, 2 µl of ribonuclease A (RNase A, 10 mg/ml) and 100 µl of Actinase E (20 mg/ml) were added. After a resuspension procedure, 1.5 ml of SE solution was added, and the mixture was incubated at 37°C for 2 hours and then diluted with 1 ml of TE solution.

Then, an extraction procedure with a phenol-chloroform solution was repeated until no more protein layer appeared, finally followed by chloroform extraction. The extract was treated with a 1/20 volume of 5 M sodium chloride and 3 volumes of cold ethanol to cause precipitation. The thus-obtained DNA was washed with 70% ethanol. The genomic DNA obtained was dissolved in TE solution and assayed by measuring the absorbance at 260 nm (spectrophotometer: Shimadzu UV-160).

### (2) Southern blot analysis using CYP2D6 cDNA

The genomic DNA obtained above in (1) (8 µg) was digested with the restriction enzyme EcoRI or XbaI and electrophoresed on a 0.8% agarose gel in a pulsed field (using Toyo's Elepos PS-1510; 100 V, 2 seconds forward, 0.6 second backward), followed by denaturation with 0.5 N sodium hydroxide-1 M sodium chloride solution and 1 M Tris-HCl (pH 7.4)-1 M sodium chloride solution, each time with 30 minutes of neutralization. The gel was immersed in 20 x SSC for 30 minutes and DNA was transferred to a nylon membrane (Schleicher & Shuell) over 8 to 12 hours by the capillary method. The membrane was washed with 3 x SSC and baked at 80°C for 2 hours to cause fixation of DNA to the nylone membrane.

The nylon membrane was blocked by incubating in prehybridization buffer (pH 8.0) (containing 1 x hybridization buffer and 1 x Denhardt's solution) containing 100 µg/mg of heat-denatured salmon sperm DNA at 65°C for 2 hour, then 50 ng of a probe (CYP2D6 cDNA having a total length of 1.5 kb) labeled with [α-³²P]dCTP (Amersham) in prehybridization buffer (pH 8.0) containing 100 µg/mg of heat-denatured salmon sperm DNA and then heat-denatured was added, and hybridization was performed at 65°C for 8 to 12 hours. After hybridization, the nylon membrane was washed with one portion of 2 x SSC-0.1% SDS at room temperature for 15 minutes and one portion of 0.5 x SSC-0.1% SDS at 50°C for 15 minutes, then air-dried and subjected to autoradiography at -80°C for 12 to 16 hours.

### (3) Results of analysis and discussion

Peripheral blood samples were taken from PMs showing a log₁₀ metabolic ratio (MR = unchanged drug/hydroxylated form metabolite; a PM is defined as a subject with log₁₀MR > 1.1) of 1.50 in human metabolism testing using the antihypertensive debrisoquin and their parents as well as extensive metabolizers (hereinafter abbreviated as EMs), and genomic DNA samples were prepared according to the procedure mentioned above under (1).

Each genomic DNA thus prepared was treated with the restriction enzyme EcoRI or XbaI according to the procedure mentioned above under (2) and subjected to Southern blot analysis.

As a result, in the analysis for CYP2D6 of the EcoRI-treated samples from PMs and their parents, bands of 16.0, 9.4 and 8.6 kb were confirmed in common.

Meanwhile, two pseudogenes CYP2D7 and CYP2D8 very highly homologous in base sequence to CYP2D6 have been reported and it is known that, according to Southern blot analysis after EcoRI treatment, CYP2D6 and these pseudogenes give bands of 9.4, 16.0 and 8.6 kb, respectively [cf. e.g. Kimura, S. et al., op. cit.].

Similarly, analysis of the samples mentioned above after XbaI treatment gave, commonly in three subjects, bands of 29.0 and 4.8 kb while, in PMs and their fathers, an additional band was observed at 11.5 kb.

A number of investigations so far made concerning the above-mentioned Southern blot analysis following XbaI treatment indicate gene polymorphism in which bands of 44.0, 29.0, 11.5 and 16.0 + 9.0 kb are involved. According to these reports, the 29.0 kb band is detected when three CYP2D genes are located in the order CYP2D8, CYP2D7 and CYP2D6 from the 5' side, and the 44.0 kb band is detected when one more CYP2D7 is added to the above three genes and they are located in the order CYP2D8, CYP2D7, CYP2D7 and CYP2D6 from the 5' side. It is also reported that the 11.5 kb band indicates complete deficiency of CYP2D6 (type D mutation) [Johansson, I., Yue, Q. Y., Dahl, M. L., Heim, M., Bertilsson, L., Meyer, U. A., Sjoqvist, F. and Ingelman-Sundberg, M., Eur. J. Clin. Pharmacol., 40, 553-556 (1991)] and that when the 16.0 and 9.0 kb bands are simultaneously detected, there is a genomic DNA mutation between CYP2D7 and CYP2D6.

In view of the foregoing, it was supposed that PMs have the type D mutation in which one of the alleles is completely deficient in CYP2D6 and which is inherited from their fathers.

### Reference Example 2

### Search for mutation of CYP2D6 gene by PCR analysis

### (1) Primer designing or selection

For detecting the type A mutation and type B mutation in the CYP2D6 gene, primers were designed such that these mutation sites of the CYP2D6 gene alone are specifically amplified. Further, for judging of the type A mutation, two primers (A'-SW and A'-SM) were designed by allocating the mutation site to the 3' terminus with a difference by one base for selectivity.

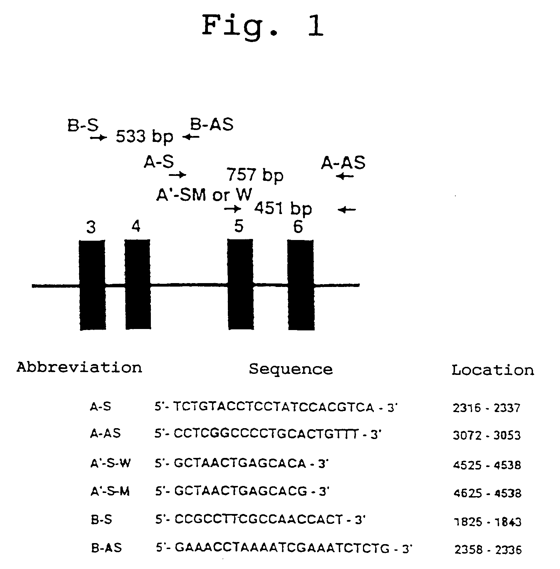

The base sequences of these primers and the sites of occurrence thereof are shown in Fig. 1.

### (2) Primer purification

The primers designed or selected as mentioned above in (1) were prepared and purified as follows. Thus, each oligonucleotide synthesized and detritylated with 3 ml of 29% ammonia water was cut off from the resin with 3 ml of 29% ammonia water and incubated at 60°C for 4 hours. The ammonia water was evaporated, the pellet obtained was dissolved in 20 µl of formamide dye, and the solution was heated at 100°C for 5 minutes and then subjected to 20% denatured acrylamide gel electrophoresis. The buffer solution used for the electrophoresis was 1 x TBE.

After electrophoresis, the oligonucleotide-containing gel portion was located by UV irradiation and then excised, extruded using a 1-ml syringe and crushed. STE (5 ml) was added threreto and extraction was effected for 10 to 12 hours with slowly rotating on a rotator. The extract was heated at 100°C for 10 minutes and then centrifuged at 3,000 rpm for 10 minutes. The supernatant was passed through a DEAE-Sephadex A-50 column equilibrated beforehand with TE buffer for thereby cause adsorption of the oligonucleotide. Elution was carried out by passing through the column 150 µl of 1.5 M sodium chloride-10 mM Tris-HCl (pH 7.5), 200 µl of 0.1 N sodium hydroxide-1 mM EDTA and 50 µl of 10 mM Tris-HCl (pH 7.5) in that order. For immediate neutralization of the eluate, 50 µl of 1 M Tris-HCl (pH 7.5) was placed beforehand in the tube for recovering the oligonucleotide. The thus-neutralized solution was subjected to ethanol precipitation, the oligonucleotide obtained was dissolved in an appropriate amount of purified water and quantitated by measuring the absorbance at 260 nm.

### (3) Genomic DNA amplification by PCR for detecting type A mutation in CYP2D6 gene

The genomic DNA containing type A mutation (deletion of one base at 2637th position in exon 5 in the genomic DNA) in the CYP2D6 gene was amplified by PCR using a DNA thermal cycler (Perkin Elmer Cetus) as follows (cf. Fig. 1).

Thus, to 1 µl of the genomic DNA solution (50 ng/µl) prepared in Reference Example 1 (1) were added 17.5 µl of purified water, 1 µl of 5 mM deoxyribonucleotide 5'-triphosphate (dNTP) solution, 2.5 µl of 10 x PCR solution (10 mM), 1 µl of 10 µM sense primer (A-S) and 1 µl of 10 µM antisense primer (A-AS). The mixture was boiled for 5 minutes and then cooled with ice for 3 minutes. To this was added 1 µl of Taq DNA polymerase (1 U/µl) to make the total volume 25 µl. After further layering of an equal volume of mineral oil thereon, the reaction was conducted by cycling at 94°C for 1 minute, 55°C for 2 minutes and 72°C for 2 minutes over 25 cycles.

After completion of the reaction, the mineral oil was removed, phenol extraction, chloroform extraction and ethanol precipitation were carried out in that order, and the DNA fragment obtained was dissolved in 30 µl of TE.

The DNA fragment amplified was identified as the CYP2D6 gene based on its band length (757 bp) and by restriction enzyme (AvaI and PvuII) treatment, followed by observation of 350, 204 and 203 bp bands after AvaI treatment and of 464 and 295 bp bands after PvuII treatment.

If, in the above procedure, the CYP2D7 gene is amplified, bands are observed at 400 and 350 bp upon AvaI treatment and, if CYP2D8 is amplified, bands are observed at 431, 146, 90, 70 and 27 bp upon PvuII treatment, so that these can be clearly distinguished from the above-mentioned CYP2D6.

### (4) Judgment about type A mutation in CYP2D6 gene

Judgment was made about the type A mutation in the CYP2D6 gene using the two sense primers (A'-SW and A'-SM) differing only by one base at the 3' terminus in combination with the antisense primer (A-AS) used in the above step (3) and using the sample solution prepared in the above step (3), as follows.

Thus, to 1 µl of the sample solution prepared in (3) were added 17.5 µl of purified water, 1 µl of 5 mM dNTP solution, 2.5 µl of 10 x PCR solution (7.5 mM), 1 µl of 10 M sense primer (A'-SW or A'-SM) for judgment and 1 µl of 10 M antisense primer (A-AS). The mixture was boiled for 5 minutes and then cooled with ice for 3 minutes. Taq DNA polymerase (1 U/µl; 1 µl) was added to make the total volume 25 µl, further an equal volume of mineral oil was layered thereon and the reaction was effected by cycling at 94°C for 1 minute, 55°C for 1 minute and 72°C for 1 minute over 25 cycles. After completion of the reaction, the mineral oil was removed and the reaction mixture was subjected to electrophoresis on a 1.5% agarose gel containing 0.02% ethidium bromide (EtBr), to make a judgment.

Of the sense primers differing only by one base at the 3' terminus, A'-SW is intended for specific amplification of the wild strain alone and A'-SM for specific amplification of the type A mutant alone, so that when a 451 bp band is observed on the A'-SW side alone, the type in question can be judged as wild type, when the 451 bp band is observed on both the A'-SW and A'-SM sides, as heterogenic type A mutation, and when the 451 bp band is observed only on the A'-SM side, as homogenic type A mutation.

As a result, the 451 band was observed only on the A'-SW side in the PM as well as in the parents. It was thus confirmed that the mutation in question was not the type A mutation.

### (5) Genomic DNA amplification by PCR for detecting type B mutation of CYP2D6 gene

The genomic DNA containing type B mutation (one base substitution at the 1934th position of the genomic DNA, i.e. at the site of exon-intron junction in exon 4) in the CYP2D6 gene was amplified in the same manner as described above in (3) using (B-S) as the sense primer and (B-AS) as the antisense primer.

The DNA fragment amplified was identified as CYP2D6 based on its band length (533 bp) and by restriction enzyme (AvaI) treatment followed by observation of 311, 146 and 76 bp bands. If, in the above procedure, CYP2D7 or CYP2D8 is amplified, bands are observed at 457 and 76 bp after treatment with AvaI, hence a clear distinction can be made between these and the above-mentioned CYP2D6.

### (6) Judgment about type B mutation in CYP2D6 gene

Judgment can be made about the type B mutation in the CYP2D6 gene by utilizing the disappearance of the recognition site for the restriction enzyme MvaI as a result of the one base substitution at the exon-intron junction site in exon 4.

Thus, to 10 µl of the sample solution prepared as described above in (5) were added 2 µl of 10 x M solution, 2 µl of MvaI (10 U/µl) and 6 µl of purified water to make the total volume 20 µl. The resulting mixture was incubated at 37°C for 2 hours and then subjected to electrophoresis using a 2% agarose gel.

When a 279 bp band is observed, the type in question can be judged as wild type, when bands are observed at 384 and 279 bp, as heterogenic type B mutation and, when a 384 bp band is observed, as homogenic type B mutation.

As a result, the 279 bp band was observed with the PM as well as with the parents. It was thus confirmed that the mutation is not the type B mutation.

### Example 1

### Analysis of CYP2D6 gene base sequence in PM

### (1) Primer designing and purification

Primers were designed so that all the exon and exon-intron junction sites of the CYP2D6 gene divided into 4 segments could be specifically amplified. The primers were each prepared and purified in the same manner as in Reference Example 2 and, using these, the respective fragments were amplified by the PCR method as follows.

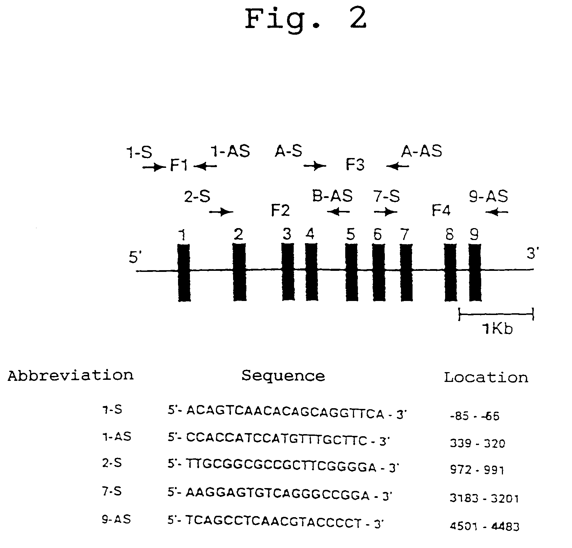

The base sequences of the primers employed, their corresponding sites on CYP2D6 and the respective fragments to be obtained are schematically shown in Fig. 2. The antisense primer (B-AS) employed in the above for amplifying the segment covering exons 2 to 4, and the sense primer (A-S) and antisense primer (A-AS) for amplifying the segment covering exons 5 to 6 were respectively the same as those used in Reference Example 2.

### (2) Amplification by PCR of exons and exon-intron junction sites of CYP2D6 gene

The genomic DNA containing the exons and exon-intron junction sites of the CYP2D6 gene was amplified using the PCR technique, as follows.

Thus, to 1 µl of the genomic DNA solution (50 ng/µl) prepared in Reference Example 1 were added 17.5 µl of purified water, 1 µl of 5 mM dNTP solution, 2.5 µl of 10 x PCR solution (10 mM), 1 µl of 10 µM sense primer and 1 µl of 10 µM antisense primer. The mixture was boiled for 5 minutes and then cooled with ice for 3 minutes. Thereto was added 1 µl of Taq DNA polymerase (1 U/µl) to make the total volume 25 µl and, after further layering of an equal volume of mineral oil thereon, the reaction was conducted by cycling at 94°C for 1 minute, 53°C (in the case of fragment F1), 48°C (F2), 55°C (F3) or 50°C (F4) for 2 minutes and 72°C for 2 minutes over 25 cycles. After completion of the reaction, the mineral oil was removed, phenol extraction, chloroform extraction and ethanol precipitation were carried out in that order, and each DNA fragment obtained was dissolved in 20 µl of TE.

That the DNA fragments amplified were respectively products originating from CYP2D6 was confirmed based on the respective band lengths and by treating with an appropriate restriction enzyme or enzymes (F1: HhaI; F2: AvaI; F3: AvaI, PvuII; F4: StyI), followed by band observation.

### (3) Subcloning into BlueScript vector

Each DNA fragment (F1 ∼ F4) of CYP2D6 obtained as described above in (2) was rendered blunt-ended as follows.

Thus, an amount equivalent to 500 ng of each fragment was dissolved in 20 µl of TE, the total volume was made 40 µl by adding 8 µl of 2 mM dNTP, 1 µl of T4 DNA polymerase, 4 µl of 10 x T4 DNA polymerase buffer (pH 8.8) and 7 µl of purified water, and the reaction was carried out at 37°C for 15 minutes. The total volume was made 200 µl by adding purified water, then phenol extraction (twice) and chloroform extraction (once) were carried out, 15 µl of 3 M sodium acetate solution (pH 5.6) and 375 µl of cold ethanol were added to the supernatant obtained, the resulting mixture was cooled to -80°C and centrifuged at 12,000 rpm and 4°C for 20 minutes, and the sediment was washed with 70% ethanol and then dried.

Since the above PCR products were phosphate group-free at the 5' terminus, phosphorylation was carried out as follows. Thus, 75 µl of denaturation buffer (pH 9.5) was added to each blunt-ended DNA fragment, and the mixture was boiled for 2 minutes and then allowed to stand on ice for 2 minutes. The total volume was made 100 µl by adding 1 µl of adenosine triphosphate (ATP, 50 picomoles/µl), 10 µl of 10 x blunt end buffer, 12 µl of purified water and 2 µl of T4 polynucleotide kinase (11 U/µl), and the reaction was conducted at 37°C for 1 hour. The total volume was made 200 µl by adding purified water, then phenol extraction (twice) and chloroform extraction (once) were carried out, the supernatant obtained was supplemented with 15 µl of 3 M sodium acetate (pH 5.6) and 375 µl of cold ethanol, the mixture was cooled to -80°C and then centrifuged at 12,000 rpm and 4°C for 20 minutes, and the sediment was washed with 70% ethanol. The sediment was dried and dissolved in 10 µl of purified water. In this way, the respective insert fragments were prepared.

BlueScript vector (2 µg) was treated with HincII and then the total volume was made 52 µl by adding 47 µl of 1 M Tris-HCl (pH 8.0) and 2 µl of alkaline phosphatase (0.5 U/µl), and the reaction was carried out at 65°C for 30 minutes. The total volume was made 200 µl by adding purified water and then phenol extraction (three times) and chloroform extraction (once) were carried out. To the supernatant were added 15 µl of 3 M sodium acetate (pH 5.6) and 375 µl of cold ethanol, the mixture was cooled to -80°C and centrifuged at 12,000 rpm and 4°C for 20 minutes, and the sediment was washed with 70% ethanol, then dried and dissolved in 20 µl of TE. In this way, dephosphorylation was effected on the vector side.

Each CYP2D6 exon- and exon-intron junction site-containing fragment (100 ng) was mixed with 10 ng of BlueScript vector and ligation was carried out. After completion of the ligation reaction, 300 µl of a suspension of competent cells was added to the DNA solution and the mixture was allowed to stand on ice for 20 minutes. Heat shock was given thereto at 42°C over 2 minutes, then 1 ml of LB solution was added, and the mixture was incubated for 1 hour, followed by 3 minutes of centrifugation at 3,000 rpm and 4°C. The supernatant (1 ml) was removed, the sediment was again suspended and used for seeding LB medium therewith, followed by overnight incubation at 37°C to give transformants.

The host Escherichia coli strain used was TG1 and the competent cells mentioned above were prepared in the following manner.

LB medium (200 ml) was sowed with 2 ml of an overnight culture of the Escherichia coli strain and cultivation was carried out at 37°C until the absorbance at 650 nm amounted to about 0.6. The culture was cooled in ice water for 30 minutes and then cells were harvested and suspended in 100 ml of 50 mM sodium calcium. After 15 minutes of agitation by slow inversion, the suspension was allowed to stand on ice for 1 hour. Cells were then again collected and suspended in 20 ml of 50 mM calcium chloride-20% glycerol. The suspension was distributed in 310-µl portions into tubes, immediately frozen using liquefied nitrogen and stored at -80°C. All the procedural steps after cell harvesting were carried out in a low temperature room (4°C).

### (4) Small quantity preparation of double-stranded DNA

A transformant colony obtained in the above manner was recovered and cultured in 3 ml of LBAmp⁺ medium. Cells were recovered by centrifuging this culture fluid and small quantity preparation of the plasmid DNA was carried out in the following manner.

Thus, 1.5 ml of the above culture fluid was transferred to a tube and centrifuged at 5,000 rpm for 5 minutes, the cell sediment was recovered and suspended in 200 µl of 10 mM EDTA solution, 400 µl of 1% SDS-0.1 M sodium hydroxide solution was added, and the mixture was allowed to stand on ice for 5 minutes. After addition of 200 µl of 5 M potassium acetate, the resultant mixture was cooled on ice for 5 minutes and then centrifuged at 13,000 rpm for 5 minutes. The supernatant was transferred to another tube and 700 µl of isopropanol was added. The mixture was centrifuged at 13,000 rpm and 4°C for 5 minutes, the sediment obtained was suspended in 100 µl of 20 mM Tris-HCl (pH 7.5)/10 mM EDTA solution, 1 µl of RNase I (10 mg/ml) was added and the mixture was incubated at 65°C for 15 minutes. After phenol extraction and chloroform extraction, 12 µl of 3 M sodium acetate (pH 5.6) and 224 µl of cold ethanol were added, the mixture was cooled to -80°C and then centrifuged at 13,000 rpm and 4°C for 10 minutes, and the sediment was washed with 70% ethanol. After drying, the sediment was dissolved in 20 µl of TE. By subjecting the plasmids recovered to treatment with restriction enzymes selected based on the multicloning site of the vector and the restriction enzyme map of the insert DNA fragment, followed by agarose gel electrophoresis, it was confirmed that insertion of the respective DNA fragments had been achieved as desired.

### (5) Subcloning into M13 vector

The direction of the insert DNA fragment in the plasmid was confirmed by treatment with restriction enzymes selected on the basis of the multicloning site of the vector and the restriction enzyme map of the insert DNA fragment.

Ligation was carried out for inserting the fragments recovered after treatment with two restriction enzymes (EcoRI and BamHI) having their recognition sites only in the multicloning site of the vector into the M13 vector treated in the same manner as these fragments. To 30 µl of each DNA solution (100 ng) obtained after completion of the ligation reaction was added 300 µl of a suspension of competent cells, and the mixture was allowed to stand on ice for 40 minutes. After giving heat shock at 42°C for 2 minutes, 3 ml of H top agar was mixed with 200 µl of an overnight culture of Escherichia coli and the mixture was spread on H agar medium, followed by overnight incubation at 37°C to give transformants. The host Escherichia coli strain used was JM103. The competent cells were prepared by the procedure mentioned above in (4).

### (6) Preparation of single-stranded phage DNA

Single-stranded phage DNA preparation was carried out as follows. Thus, a transformant plaque was recovered and cultivated in 3 ml of 2 x TY medium for 6 hours. A 1.5-ml portion of this culture fluid was centrifuged at 15,000 rpm for 5 minutes (twice) and the supernatant was recovered. To 1.3 ml of the supernatant was added 200 µl of 20% polyethylene glycol (PEG)-2.5 M sodium chloride. The mixture was allowed to stand at room temperature for 15 minutes and then centrifuged at 15,000 rpm for 5 minutes to cause the phage DNA to precipitate. After 2 minutes of further centrifugation at 15,000 rpm, the supernatant was completely removed, the sediment was dissolved in 200 µl of TE, 100 µl of phenol was added and, after stirring, the mixture was allowed to stand at room temperature for 15 minutes. Further, 100 µl of chloroform was added and the same procedure was performed, followed by 5 minutes of centrifugation at 10,000 rpm. A 150-µl portion of the supernatant was taken, 15 µl of 3 M sodium acetate (pH 5.6) solution and 375 µl of cold ethanol were added, and the mixture was cooled to -80°C for ethanol precipitation. Centrifugation was performed at 12,000 rpm and 4°C for 20 minutes and the sediment was washed with 70% ethanol. The sediment was dried and dissolved in 20 µl of TE for recovering a single-stranded DNA. Agarose gel electrophoresis was performed and, by using, as a control, the single-stranded DNA prepared from the M13 vector free of any fragment and based on the fact that a band of low mobility was detected, it was confirmed that the fragment had been inserted as intended.

### (7) Analysis of base sequences of exons and exon-intron junction sites of CYP2D6 gene

Base sequence analysis was performed using the single-stranded DNA obtained as described above in (6) by the method described in the literature [Sanger, F., Miklen, S. and Coulson, A. R., Proc. Natl. Acad. Sci., U.S.A., 74, 5463-5467 (1977)], as follows. The sequencing reaction was carried out according to the manual attached to Sequenase™ VER. 2.0.

Thus, first, 7 µl of the template DNA (700 ng), 1 µl of the primer (1 picomole/µl) and 2 µl of 5 x annealing buffer (pH 7.5) were mixed, the mixture was heated at 65°C for 2 minutes and then gradually cooled for annealing of the primer with the template DNA. Then, 1 µl of 0.1 M DTT, 2.0 µl of labeling mix, 0.5 µl of [α-³³P]deoxyadenosine 5'-triphosphate (dATP) and 2 µl of 8-fold diluted Sequenase were added, and the mixture was incubated at room temperature for 2 to 5 minutes. During that time, the reaction terminator solution containing the four dideoxyNTPs was distributed in 2.5 µl portions into tubes and preincubated at 37°C. After completion of the reaction, the reaction mixtures were respectively added in 3.5 µl portions to the termination solutions, followed by 5 minutes of further incubation at 37°C. Formamide dye solution (4 µl) was added and each mixture was boiled for 2 minutes and then applied to a gel. The gel was a sucrose density gradient gel with a gel thickness of 0.4 mm prepared by using a 60 cm glass plate. To 40 ml of gel A solution and 15 ml of gel B solution were added 32 µl of 20% N,N,N',N'-tetramethylethylenediamine (TEMED) and 9 µl of 10% ammonium persulfate (APS), respectively. The gel A solution and gel B solution were drawn, in that order, into a 50-ml disposable syringe and gently stirred by sucking 2 or 3 bubbles, and the mixture was poured into the glass plate. The remaining space was filled with the top gel. Electrophoresis was performed at a constant voltage of 2,500 to 3,000 V for 2 to 7 hours using 1 x TBE. Thereafter, the gel was immersed in 10% acetic acid-10% methanol for 15 minutes for DNA fixation and then dried under suction using a gel drier. An X-ray film was exposed thereto overnight at -80°C.

### (8) Results of analysis and discussion

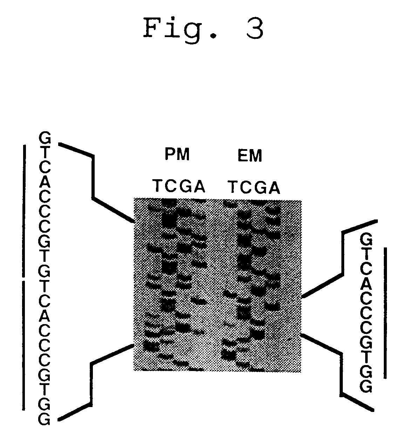

As a result of analysis of the base sequence of each of the PM-derived exons and exon-intron junction sites, insertion of a repetition of the 9-base sequence TCACCCGT (4213-4222 bp) in exon 9 (4213 bp) was established (Fig. 3).

It was deduced that this insertion results in insertion of three amino acids (valine, proline, threonine). This site was located immediately behind the hem binding region (HR-2 region), which is an iron binding site important in drug oxidation reactions and has an important role in the hem protein P450. It was surmised that the insertion of three amino acids resulting from said base insertion exert some influence on the metabolic activity in PMs, remarkably decreasing the metabolic ability.

The above-mentioned mutation in the CYP2D6 gene is a novel type of mutation not yet reported so far and this mutation was considered as a cause factor to PMs.

### Example 2

### Detection of novel mutation in CYP2D6 gene (PCR-RFLP)

### (1) Primer designing and purification

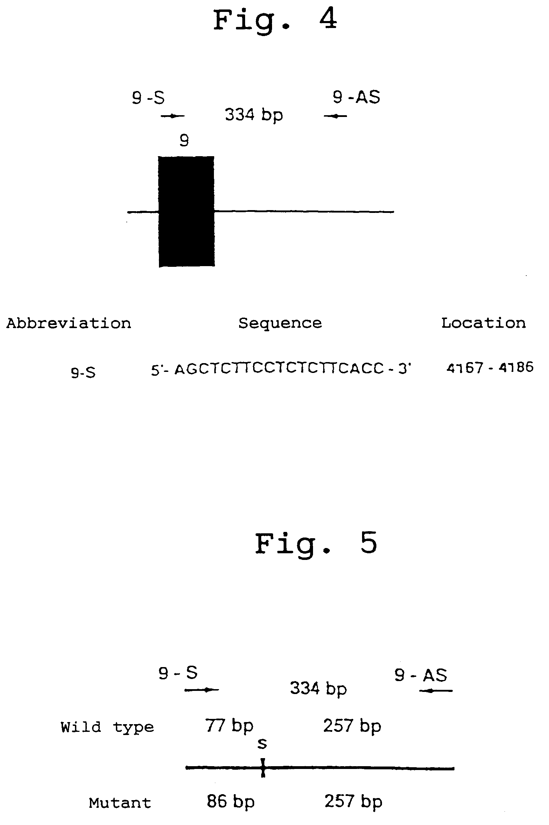

A sense primer was designed such that it contains the insertion site, which is the novel mutation site, in exon 9 and allows specific amplication of CYP2D6 alone. The antisense primer used was the same as that constructed in Example 1 (9-AS). The primers selected and their locations are shown in Fig. 4.

Each primer was purified in the same manner as in Reference Example 2. Using these primers, the genomic DNA of exon 9 was amplified by the following PCR method.

### (2) Amplification of exon 9 of CYP2D6 gene by PCR

To 1 µl of the genomic DNA solution (50 ng/µl) prepared in Reference Example 1 were added 17.5 µl of purified water, 1 µl of 5 mM dNTP solution, 2.5 µl of 10 x PCR solution [in this example alone, its composition was: 200 mM Tris-HCl (pH 8.4), 250 mM potassium chloride, 0.5% Tween 20, 1 mg/ml gelatin, 10 mM magnesium chloride], 1 µl of 10 µM sense primer (9-S) and 1 µl of 10 µM antisense primer (9-AS). The mixture was boiled for 5 minutes and then cooled on ice for 3 minutes. To this was added 1 µl of Taq DNA polymerase (1 U/µl) to make the total volume 25 µl and, after further layering of an equal volume of mineral oil thereon, the reaction was carried out by cycling at 94°C for 1 minute, 55°C for 1 minute and 72°C for 2 minutes over 25 cycles.

After the reaction, the mineral oil was removed, phenol extraction, chloroform extraction and ethanol precipitation were performed in that order, and the DNA fragment obtained was dissolved in 20 µl of TE.

That the DNA fragment amplified was derived from CYP2D6 was confirmed on the basis of its band length (334 bp) and observation, following restriction enzyme (StyI, Asp718) treatment, of a 257 bp band and a 77 bp band upon StyI treatment and of a 189 bp band and a 145 bp band upon Asp718 treatment. If, on that occasion, CYP2D7 is amplified, a 331 bp band will be observed after StyI treatment and if CYP2D8 is amplified, a 320 bp band will be observed after Asp718 treatment; thus, clear differentiation from these is possible.

### (3) Judgment about insertion in exon 9 of CYP2D6 gene by restriction enzyme treatment of PCR products

The judgment was made by treating the DNA fragment amplified above in (2) with the restriction enzyme StyI followed by band length determination (Fig. 5).

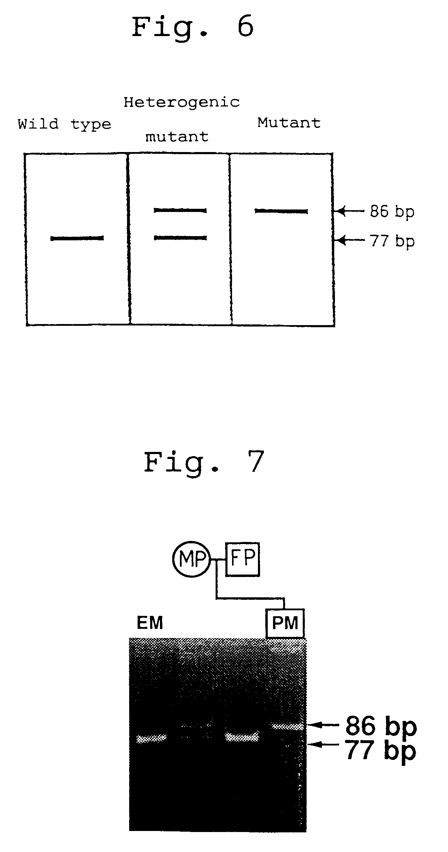

Thus, to 10 µl of the sample solution prepared in (2) were added 2 µl of 10 x H solution, 2 µl of StyI (10 U/µl) and 6 µl of purified water to make the total volume 20 µl. The resulting mixture was incubated at 37°C for 2 hours. After the reaction, phenol extraction, chloroform extraction and ethanol precipitation were performed in that order, and the DNA fragment was dissolved in 5 µl of TE and subjected to electrophoresis using a 10% acrylamide gel. When a 77 bp band was observed, the DNA in question was judged as wild type, when the 77 bp band and a 86 bp band were observed, as heterogenic mutant and, when a 886 bp band was observed, as homogenic mutant (Fig. 6).

### (4) Results and discussion

The results of testing a PM and the parents of the PM for detecting the above-mentioned novel mutation in the CYP2D6 gene are shown in Fig. 7. The 86 bp band was observed in the PM and the mother (in the figure, MP) and the mutation (insertion) concerned with the present invention was established thereby. This mutation in the MP thus proved to have been inherited from the mother. In the figure, FP indicates the father of the PM and the result for an EM is also shown in the figure.

It is apparent from the above results that the detection of a novel mutant of the CYP2D6 gene according to the present invention is useful for the genetic diagnosis of PM.

### INDUSTRIAL APPLICABILITY

In accordance with the present invention, a novel mutation is revealed in the CYP2D6 gene and a method of detecting a novel CYP2D6 gene polymorph containing such mutation is provided.

By detecting said CYP2D6 gene polymorph, gene diagnosis becomes possible in PMs, in particular in PMs in whom the causative factor is other than the known CYP2D6 gene polymorphs. Thus, said detection method is very useful, for example, in diagnosing, predicting or investigating causes of adverse effects of various drug substances.

## Claims

1. A method of detecting a polymorph of the human cytochrome P450 2D6 gene which comprises detecting the occurrence, in exon 9 of the human cytochrome P450 2D6 gene, of an insertion of a repetition of the 9 bases TCACCCGTG.
